# EUROPEAN PATENT SPECIFICATION

(11) **EP 2 925 257 B1**
(45) Date of publication and mention of the grant of the patent: **31.01.2018**
(21) Application number: 13857790.3
(22) Date of filing: 27.11.2013
(51) Int. Cl.: A61F 2/01

(54) **PERCUTANEOUS TRANSLUMINAL ANGIOPLASTY DEVICE WITH INTEGRAL EMBOLIC FILTER**
PERKUTANE TRANSLUMINALE ANGIOPLASTIEVORRICHTUNG MIT INTEGRIERTEM EMBOLIEFILTER
DISPOSITIF D'ANGIOPLASTIE TRANSLUMINALE PERCUTANÉE AVEC FILTRE EMBOLIQUE INTÉGRÉ

(30) Priority: 27.11.2012 US 201261730213 P
(43) Date of publication of application: 07.10.2015
(73) Proprietor: Contego Medical, LLC, Raleigh NC 27603 (US)
(72) Inventor: PATEL, Udayan G., San Jose, CA 95120 (US); SACHAR, Ravish, Raleigh, NC 27612 (US)
(74) Representative: Hoefer & Partner Patentanwälte mbB
(86) International application number: PCT/US2013/072232
(87) International publication number: WO 2014/085590

(56) References cited:
- US-A1- 2003 060 843
- US-A1- 2003 220 665
- US-A1- 2004 167 564
- US-A1- 2005 228 438
- US-A1- 2010 010 534
- US-A1- 2010 106 182
- US-A1- 2010 106 182
- US-A1- 2011 137 399

## Description

### CROSS-REFERENCE TO RELATED APPLICATION

This application claims priority to U.S. provisional application Ser. No. 61/730,213 filed on November 27, 2012.

### BACKGROUND

### Field of the Invention

Implementations described herein relate generally to surgical devices and relate more specifically to percutaneous transluminal angioplasty devices.

### Related Art

The vascular bed supplies a constant flow of oxygen-rich blood to the organs. In diseased vessels, blockages can develop that can reduce blood flow to the organs and cause adverse clinical symptoms up to and including fatality. Diseased vessels can comprise a range of material from early-stage thrombosis to late-stage calcified plaque.

Angioplasty can be described as a catheter-based procedure performed by a physician to open up a blocked vessel and restore blood flow. An entry site can be opened, for example, in the patient's groin, arm, or hand, and a guide wire and catheter can be advanced under fluoroscopic guidance to the location of the blockage. A catheter having a small balloon adjacent its distal end can be advanced under fluoroscopic guidance until the balloon lies within the stenosed region. The balloon can be then inflated and deflated one or more times to expand the stenosed region of the artery.

Angioplasty can release embolic particles down-stream from the stenosed location. These embolic particles can result in adverse clinical consequences. It has been shown beneficial to trap these embolic particles to prevent them from traveling downstream with blood flow to the capillary bed (e.g., Baim D S, Wahr D, George B, et al., Randomized trial of a distal embolic protection device during percutaneous intervention of saphenous vein aorto-coronary bypass grafts, Circulation 2002; 105:1285-90).

In addition to balloon angioplasty, stenoses can also be treated with stents and with mechanical thrombectomy devices. These devices can be also prone to releasing embolic particles downstream from the stenosed location.

Systems available today used to catch these embolic particles consist primarily of filter systems or occlusion balloon systems, both built on a guidewire. US patent application publication US 2010/0106182 A1 for example discloses an angioplasty device having a filter associated with it to capture embolic particles that may be broken free during an angioplasty procedure. In one disclosed embodiment the embolic filter has a frame in which struts are connected to end rings and to each other by a plurality of interconnected oval members. In another disclosed embodiment the device includes an actuator at the proximal end of an actuator wire. By rotating a knob on the device, the physician can smoothly tension the wire by a predetermined amount to prevent the filter from opening too much or too little. These systems suffer shortcomings related to simplicity of use and crossing tight lesions with a filter or balloon guidewire that can be larger in diameter than the guidewire which would normally be used. These embolic protection guidewires also suffer from flexibility and stability problems that render the protected angioplasty procedure relatively more difficult in many cases. In the case of saphenous vein grafts, the problems relate specifically to aorto-ostial lesions, where the guidewire may not be long enough to provide support, or distal vein graft lesions, where there can be not enough of a landing zone for the filter. The latter can be a problem as currently available filter systems can have a considerable distance between the treatment balloon and the distal filter. This distance can be a problem not only in distal vein graft lesions, but also in arterial stenoses in which there can be a side branch immediately after the stenosis. In such cases, the filter can often be deployed only distal to the side branch, thus leaving the side branch unprotected from embolic particles.

Accordingly, a need exists for improved percutaneous transluminal angioplasty devices having an integral embolic filter.

### SUMMARY

It is to be understood that this summary is not an extensive overview of the disclosure. This summary is exemplary and not restrictive, and it is intended to neither identify key or critical elements of the disclosure nor delineate the scope thereof. The sole purpose of this summary is to explain and exemplify certain concepts of the disclosure as an introduction to the following complete and extensive detailed description.

Stated generally, the present disclosure comprises a percutaneous transluminal angioplasty device with integral embolic filter. Because the filter can be integral with the catheter of the angioplasty device, any need to insert a separate device into the vessel can be eliminated. Further, proper placement of the angioplasty balloon can assure proper placement of the embolic filter.

Stated somewhat more specifically, the percutaneous transluminal angioplasty device of the present disclosure comprises an embolic filter mounted to the catheter shaft at a location distal to the angioplasty balloon, stent, mechanical thrombectomy device or the like. Thus, the filter can be positioned downstream from the blockage in order to capture embolic particles that may be set loose into the blood stream during the angioplasty procedure. The embolic filter can be un-deployed against the catheter shaft in an un-deployed position to facilitate introduction and withdrawal of the device to and from the operative site. Once the angioplasty balloon, stent, mechanical thrombectomy or like device is properly positioned, means operatively associated with the embolic filter can be actuated to erect the filter to position a filter mesh across the lumen of the coronary artery.

In some aspects, the means for deploying the filter can comprise a balloon which longitudinally displaces one end of the filter toward the other, causing longitudinal ribs to bow outward, thus deploying the filter mesh. In other aspects the means for deploying the filter comprises a balloon interposed within the proximal and distal ends of the filter, whereby inflating the balloon will bias the ribs away from the catheter shaft, causing the ribs to bow outwardly to erect the filter mesh. In still other aspects the means for deploying the filter comprises a pull wire attached to one end of the filter, such that pulling on the wire longitudinally displaces one end of the filter toward the other, causing longitudinal ribs to bow outward, thus deploying the filter mesh.

In yet other aspects of the invention, a reservoir can be provided at the distal tip of the filter so that when the device collapses for withdrawal, debris does not get pushed out of the filter.

Additional features and advantages of exemplary implementations of the disclosure will be set forth in the description which follows, and in part will be obvious from the description, or may be learned by the practice of such exemplary implementations. The features and advantages of such implementations may be realized and obtained by means of the instruments and combinations particularly pointed out in the appended claims. These and other features will become more fully apparent from the following description and appended claims, or may be learned by the practice of such exemplary implementations as set forth hereinafter.

### BRIEF DESCRIPTION OF THE DRAWINGS

The accompanying drawings, which are incorporated in and constitute a part of this specification, illustrate aspects and together with the description, serve to explain the principles of the methods and systems.
FIG. **1** illustrates a partial cut away side view of a percutaneous transluminal angioplasty device, with the angioplasty balloon and embolism filter in their un-deployed positions.
FIG. **2** illustrates a partial cut away side view of the percutaneous transluminal angioplasty device of FIG. **1** showing the angioplasty balloon and embolism filter in their deployed positions.
FIG. **3** illustrates a cross sectional view taken along line **3-3** of FIG. **1****.**
FIG. **4** illustrates a cross sectional view taken along line **4-4** of FIG. **1****.**
FIG. **5** illustrates a cross sectional view taken along line **5-5** of FIG. **1****.**
FIG. **6** illustrates a second aspect of a percutaneous transluminal angioplasty device, which differs from the percutaneous transluminal angioplasty of FIGS. **1** and **2** in that the actuation balloon can be on the proximal side of the embolic filter, and the filter deploys from a different direction.
FIG. **7** illustrates a view of the percutaneous transluminal angioplasty device of FIG. **6** showing the angioplasty balloon inflated and the embolic filter deployed.
FIG. **8** illustrates a third aspect of a percutaneous transluminal angioplasty device and differs from the previously described aspects in that the means for deploying the embolic filter can be a bellows. FIG. **8** shows the angioplasty balloon and the embolic filter in their un-deployed positions.
FIG. **9** illustrates another view of the percutaneous transluminal angioplasty device of FIG. **8** showing the angioplasty balloon and the embolic filter in their deployed positions.
FIG. **10** illustrates another aspect of a percutaneous transluminal angioplasty device according to the present disclosure which employs a bellows to raise and lower the embolic filter. The aspect of FIG. **10** differs from the aspect of FIG**S**. **8** and **9** in that the bellows can be disposed on the distal end of the filter such that the filter deploys from the opposite direction. FIG. **10** shows the angioplasty balloon and the embolic filter in their un-deployed positions.
FIG. **11** illustrates another view of the percutaneous transluminal angioplasty device of FIG. **10****,** showing the angioplasty balloon and the embolic filter deployed.
FIG. **12** illustrates still another aspect of a percutaneous transluminal angioplasty device, in which the balloon interposed between the catheter shaft and the ribs forces the ribs upward, thereby causing the embolic filter to deploy. FIG. **12** shows the device with the angioplasty balloon and the embolic filter in their un-deployed configurations.
FIG. **13** illustrates another view of the percutaneous transluminal angioplasty device of FIG. **12****,** showing the angioplasty balloon and the embolic filter in their deployed configurations.
FIG. **14** illustrates another aspect of a percutaneous transluminal angioplasty device. This aspect differs from the aspects of FIGS. **12** and **13** in that the balloon can be located at the opposite end of the filter. Nonetheless, when inflated, the balloon forces the ribs away from the shaft and into their arcuate positions, thereby deploying the embolic filter. FIG. **14** shows the aspect with the angioplasty balloon un-deployed and the embolic filter retracted against the catheter shaft.
FIG. **15** illustrates another view of the aspect of FIG. **14****,** showing the angioplasty balloon inflated and the embolic filter deployed.
FIG. **16** illustrates a percutaneous transluminal angioplasty device according to the present invention. This aspect employs a pull wire operable from outside the patient which can be attached to a distal ring of the embolic filter. When the physician exerts tension on the wire, the distal ring can be displaced proximally, bringing it closer to the proximal ring, causing the ribs to bow outward and thereby deploying the embolic mesh filter. FIG. **16** shows the device with the angioplasty balloon deflated and the embolic filter un-deployed against the catheter shaft.
FIG. **17** illustrates a different view of the aspect of FIG. 16 and shows the angioplasty balloon inflated and the embolic filter deployed.
FIG. **18** illustrates another aspect of a percutaneous transluminal angioplasty device according to the present invention, showing the angioplasty balloon and the embolic filter in their un-deployed conditions. Optionally, the disclosed embolic filter can be formed from a shape memory material in which the base or unstrained shape memory is in a baseline open position or a baseline closed position.
FIG. **19** illustrates another view of the aspect of FIG. **18****,** showing the angrioplasty balloon inflated and the embolic filter raised to an open position.
FIG. **20** illustrates yet another aspect of a percutaneous transluminal angioplasty device according to the present invention, showing the angioplasty balloon and the embolic filter in their un-deployed configurations.
FIG. **21** illustrates another view of the aspect of FIG. **20****,** showing the angioplasty balloon inflated and the embolic filter deployed.
FIG. **22** illustrates a side cut away view of a coronary artery with a stenosis.
FIG. **23** illustrates the coronary artery of FIG. **20** with a guide wire fed through the coronary artery and through the stenosis.
FIG. **24** illustrates the device of FIG. **1** threaded over the guide wire of FIG. **23** and positioned such that the angioplasty balloon can be located within the stenosis.
FIG. **25** illustrates the angioplasty balloon in its deployed configuration to reduce the stenosis, and the embolic filter deployed to capture any embolic particles that may break loose into the blood stream as a result of the angioplasty procedure.
FIG. **26** illustrates a partial cut away side view of an aspect of a device in which the angioplasty balloon and embolism filter, shown in their un-deployed positions, can be reversed on the catheter shaft for peripheral vascular applications in which blood flows in the opposite direction.
FIG. **27** illustrates a partial cut away side view of the device of FIG. **26** showing the angioplasty balloon and embolic filter in their deployed positions.
FIG. **28** illustrates a side view of an embolism filter.
FIG. **29** illustrates a side view of the embolism filter of FIG. **28** with the inflation balloon and the embolic filter in their deployed configurations. The filter mesh is shown removed to reveal interior detail.
FIG. **30** illustrates a side view of the embolic filter of FIG. **28** with the inflation balloon deflated. The filter mesh is shown removed to reveal interior detail.
FIG. **31** illustrates a side view of the embolic filter of FIG. **28** being retracted into the forward end of a catheter to collapse the filter. The filter mesh is shown removed to reveal interior detail.
FIG. **32** illustrates a side view of the embolic filter of FIG. **28** with the filter deployed and the filter mesh shown.
FIG. **33** illustrates a side cutaway view of another aspect of an angioplasty device showing an angioplasty balloon and an embolic filter in their un-deployed configurations.
FIG. **34** illustrates a side cutaway view of the angioplasty device of FIG. **33** showing the angioplasty balloon and the embolic filter deployed.
FIG. **35** illustrates a side view of a further aspect of an angioplasty device in which the filter mesh extends beyond the end of the ribs so as to form a sac when the frame is in an un-deployed configuration.
FIG. **36** illustrates a side view of the angioplasty device of FIG. **35** when the frame is in an un-deployed configuration.
FIG. **37** illustrates a generally cylindrical filter frame shown unrolled and flattened.
FIG. **38** illustrates the generally cylindrical filter frame of FIG. **37** shown in a deployed configuration without the filter membrane.
FIG. **39** illustrates the generally cylindrical filter frame of FIG. **37** shown in a deployed configuration with the filter membrane.
FIG. **40** illustrates an alternate frame design that is generally cylindrical shown unrolled and flattened.
FIG. **41** illustrates another alternate frame design that is generally cylindrical shown unrolled and flattened.
FIG. **42** illustrates a top view an aspect of a filter membrane formed in the shape of a funnel.
FIG. **43** illustrates a side view of an alternate frame design that is generally cylindrical shown unrolled and flattened.
Fig. **44** illustrates a perspective view of the filter membrane of Fig. **42** and **43****.**
FIG. **45** illustrates a perspective view of an alternate frame design shown unrolled and flattened that is generally cylindrical when formed.
FIG. **46** illustrates the alternate frame design of Fig. **45** shown stretched out over a mandrel to form a substantially cylindrical shape.
FIG. **47** illustrates the alternate frame design of Fig. **45** showing the respective ends of the frame being trimmed to form a desired elongate longitudinal length. The trimmed cylindrically shaped frame design can be heat treated to set the shape memory in the formed position or, optionally, the trimmed frame can be compressed to a desired dimension and then heat treated to set the shape memory in the formed position (baseline closed position) or it can be expanded to a desired dimension and then heat treated to set the shape memory in the formed position (baseline open position).
FIG. **48** illustrates the alternate frame design of Fig. **45** showing the trimmed cylindrically shaped frame design heat treated to set the shape memory in the baseline closed position upon application of an axial compression, for example and not meant to be limiting, by an external operator pulling on a pull wire to controllably cause the mid-portion of the trimmed cylindrically shaped frame design to expand to a desired diameter, which is a multiple of the original diameter of the frame design in the baseline closed position.

### DETAILED DESCRIPTION OF THE INVENTION

The present invention is disclosed in the independent claim 1 and can be understood more readily by reference to the following detailed description, examples, drawing, and claims, and their previous and following description. Insofar as the terms "invention" and/or "embodiment" are used in the following, and/or features are presented as being optional, this should be interpreted in such a way that the only protection sought is that of the invention as defined in the claims. However, before the present devices, systems, and/or methods are disclosed and described, it is to be understood that this invention is not limited to the specific devices, systems, and/or methods disclosed unless otherwise specified, as such can, of course, vary. It is also to be understood that the terminology used herein is for the purpose of describing particular aspects only and is not intended to be limiting.

The following description of the invention provided as an enabling teaching of the invention in its best, currently known aspect. To this end, those skilled in the relevant art will recognize and appreciate that many changes can be made to the various aspects of the invention described herein, while still obtaining the beneficial results described herein. It will also be apparent that some of the desired benefits described herein can be obtained by selecting some of the features described herein without utilizing other features. Accordingly, those who work in the art will recognize that many modifications and adaptations to the present invention are possible and can even be desirable in certain circumstances and are a part described herein. Thus, the following description is provided as illustrative of the principles described herein and not in limitation thereof.

Reference will be made to the drawings to describe various aspects of one or more implementations of the invention. It is to be understood that the drawings are diagrammatic and schematic representations of one or more implementations, and are not limiting of the present disclosure. Moreover, while various drawings are provided at a scale that is considered functional for one or more implementations, the drawings are not necessarily drawn to scale for all contemplated implementations. The drawings thus represent an exemplary scale, but no inference should be drawn from the drawings as to any required scale.

In the following description, numerous specific details are set forth in order to provide a thorough understanding described herein. It will be obvious, however, to one skilled in the art that the present disclosure may be practiced without these specific details. In other instances, well-known aspects of percutaneous transluminal angioplasty devices and embolic filters have not been described in particular detail in order to avoid unnecessarily obscuring aspects of the disclosed implementations.

As used in the specification and the appended claims, the singular forms "a," "an" and "the" include plural referents unless the context clearly dictates otherwise. Ranges may be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

"Optional" or "optionally" means that the subsequently described event or circumstance may or may not occur, and that the description includes instances where said event or circumstance occurs and instances where it does not.

Throughout the description and claims of this specification, the word "comprise" and variations of the word, such as "comprising" and "comprises," means "including but not limited to," and is not intended to exclude, for example, other additives, components, integers or steps. "Exemplary" means "an example of" and is not intended to convey an indication of a preferred or ideal aspect. "Such as" is not used in a restrictive sense, but for explanatory purposes.

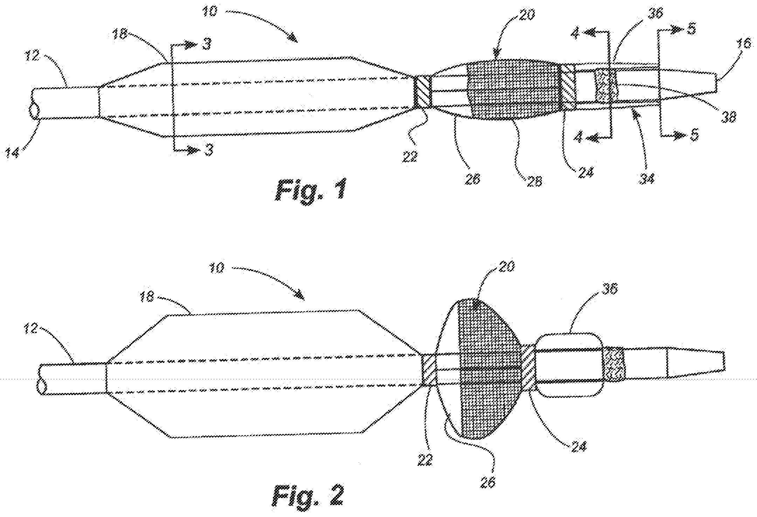

Referring now to the drawings, in which identical numbers indicate identical elements throughout the various views, Figures **1** and **2** illustrate a first aspect of a percutaneous transluminal angioplasty device **10**. The device **10** comprises an elongated catheter **12** having a shaft **14** with a proximal end (not shown) and a distal end **16.** Spaced a short distance proximally from the distal end **16** of the catheter **12** can be an angioplasty balloon **18** of conventional design. In Figure **1** the angioplasty balloon **18** can be shown in a deflated or un-deployed condition. In Figure **2** the angioplasty balloon **18** can be shown in an inflated or deployed condition.

Located between the angioplasty balloon **18** and the distal tip **16** of the catheter **12** can be a collapsible filter **20.** The filter **20** can include a proximal ring portion **22** and a distal ring portion **24.** A plurality of elongated ribs **26** extend generally longitudinally between the proximal and distal rings **22, 24.** These ribs can be made of a shape memory material, such as nitinol, and in their baseline position, these ribs can be un-deployed. A filter mesh **28** overlies the distal portion of the ribs **26.** In the aspect of Figures **1** and **2****,** the distal ring **24** can be movable toward and away from the proximal ring **22.** As the distal ring **24** moves toward the proximal ring **22,** the ribs **26** bow outward. As the ribs **26** bow outward, the filter mesh **28** overlaying the ribs can be deployed. Figure **1** shows the filter **20** in its un-deployed condition, while Figure **2** shows the filter in its deployed condition.

Means **34** can be included for deploying and collapsing the filter **20** of the device **10** shown in Figures **1** and **2****.** Specifically a balloon **36** can have its distal end **38** bonded to the shaft **14** of the catheter **12.** When the distal ring **24** is in its withdrawn position, as shown in Figure **1****,** the bulk of the balloon **36** can be folded forward over the shaft **14** of the catheter **12.** When the balloon **36** is deployed, as shown in Figure **2****,** the balloon **36** can expand proximally, pushing the distal ring **24** in a proximal direction, causing the ribs **26** to bow outward and thereby deploying the filter **20.** When the balloon **32** is deflated, the shape memory ribs can straighten, urging the distal ring **24** in a distal direction and collapsing the filter **20** to its un-deployed configuration close to the shaft **14** of the catheter **12.**

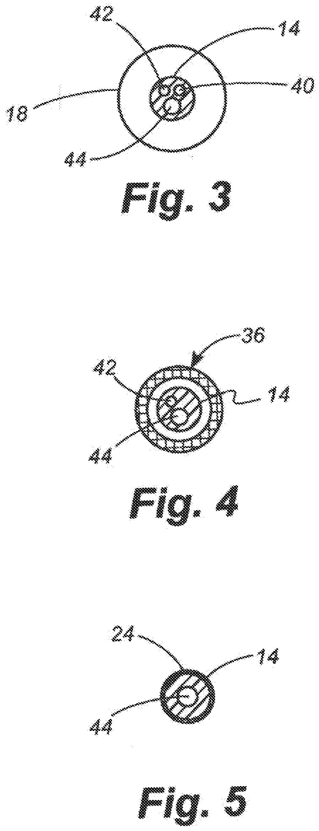

Figures **3, 4,** and **5** show cross sections of the device **10** at various locations along its length. Referring first to Figure **3****,** the catheter shaft **12** has three lumens: two smaller lumens and a large main lumen. The two smaller lumens can be inflation lumens, one lumen **40** for the angioplasty balloon **18,** and one lumen **42** for the balloon **36** which controls the filter **20.** The larger main lumen **44** can be used to receive a guide wire (not shown) over which the device **10** can be advanced to position the device for performing an angioplasty procedure.

Referring now to Figure **4****,** this cross section illustrates a location distal to the angioplasty balloon **18.** Consequently, the angioplasty balloon inflation lumen **40** has terminated and is no longer visible. Thus, Figure **4** shows only two lumens, the main lumen **44** for receiving the guide wire, and the smaller inflation lumen **42** for the filter balloon **36.**

Referring now to Figure **5****,** this cross section illustrates a location distal to the filter balloon **36,** and hence only the main lumen **44** can be visible.

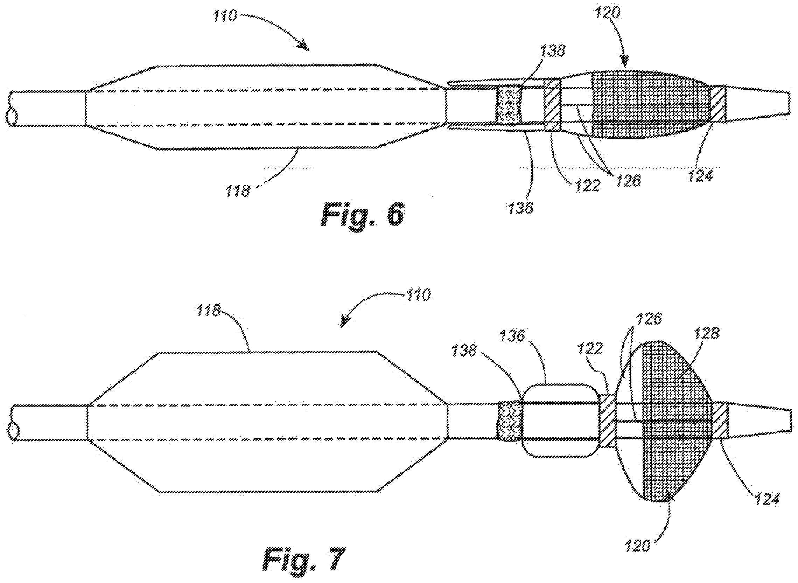

Figures **6** and **7** show an alternate percutaneous transluminal angioplasty device **110**. This device can be similar to the device **10** previously described, with the exception that the filter **120,** in this case, has its distal ring **124** fixed, and the proximal ring **122** of the filter **120** can be movable toward and away from the distal ring to cause the ribs **126** to bow outwardly or to straighten. The balloon **136** can be located on the proximal side of the filter **120** and pushes the proximal ring **122** in a distal direction when the balloon **136** can be inflated.

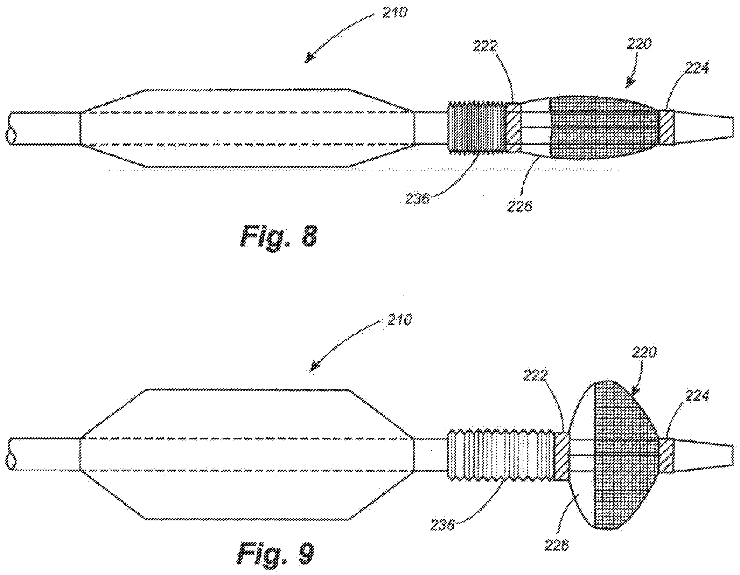

Referring now to Figures **8** and **9****,** yet another percutaneous transluminal angioplasty device **210** can be shown. This device can be similar to the device shown in Figures **1** and **2****,** with the exception that the means for deploying the filter **220** can be a bellows **236,** instead of a balloon. In Figure **8****,** the bellows **236** can be deflated and hence it can be in an un-deployed condition, permitting the ribs **226** of the filter **220** to straighten out against the shaft **214** of the catheter **212** in an un-deployed state. In Figure **9****,** the bellows **236** has been inflated, pushing the proximal ring **222** in a distal direction, bowing out the ribs **236** and deploying the filter mesh **238.**

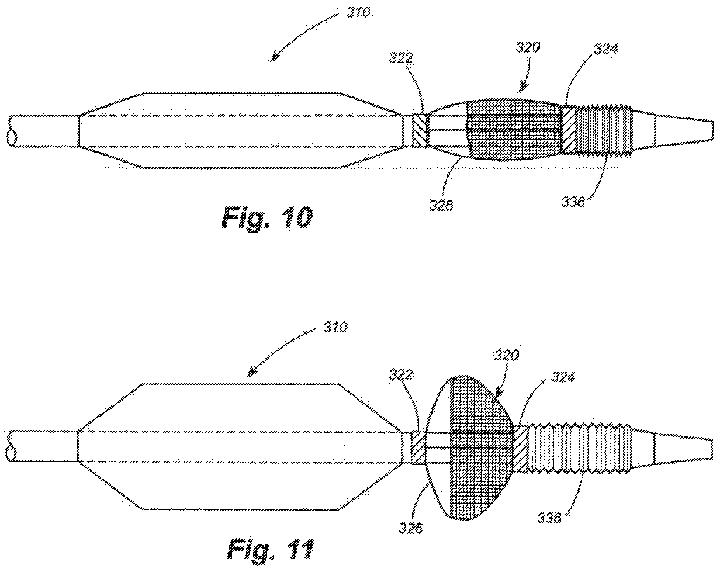

Figures **10** and **11** illustrate still another percutaneous transluminal angioplasty device **310.** This device can be similar to the device shown in Figures **8** and **9****,** with the exception that the bellows **336** can be placed on the distal side of the filter **320.** Thus, when the bellows **336** can be inflated, it moves the distal ring **324** in a proximal direction toward the proximal ring **322,** thereby causing the ribs **326** to bow outwardly, deploying the filter mesh **338.**

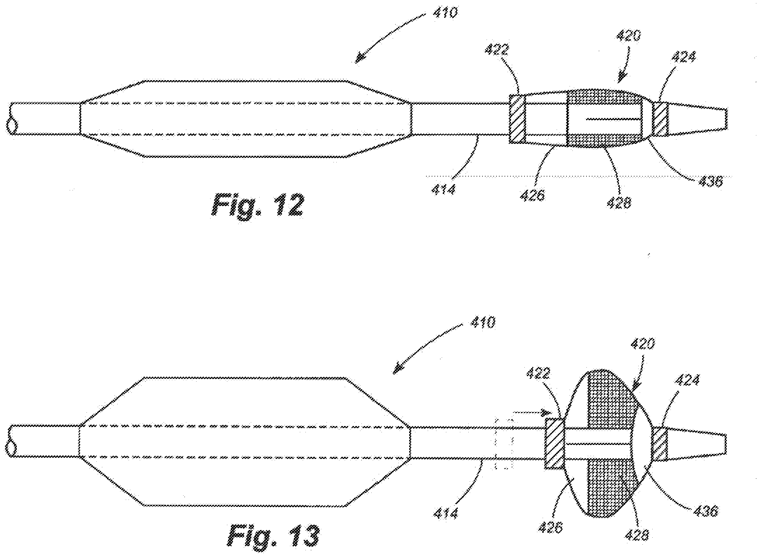

Figures. **12** and **13** depict another percutaneous transluminal angioplasty device **410.** In this device the means for deploying the filter comprises a balloon **436** disposed between the catheter shaft **414** and the ribs **426** adjacent the fixed distal ring **424** of the filter **420.** When the balloon **436** can be inflated, it forces the ribs **426** outward away from the catheter shaft **414,** thereby bowing the ribs and drawing the proximal ring **422** of the filter **420** in a distal direction. As the ribs **426** bow outward, the filter mesh **428** can be deployed, thereby raising the filter **420.**

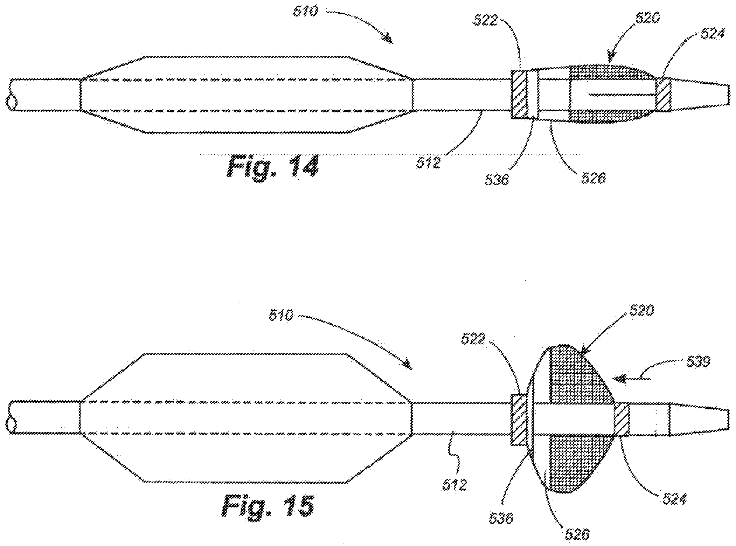

Figures **14** and **15** show a device **510** similar to that shown in FIGS. **12** and **13****,** with the exception that the balloon **536** can be placed between the catheter shaft **512** and the ribs **526** adjacent the proximal ring **522** of the filter **520.** In the device **510,** the distal ring **524** can be free to slide along the catheter shaft **512,** such that when the balloon **536** can be inflated and forces the ribs **526** to bow outward, the distal ring **524** slides in a proximal direction, as indicated by the arrow **539** as shown in Figure **15****,** causing the filter **520** to deploy.

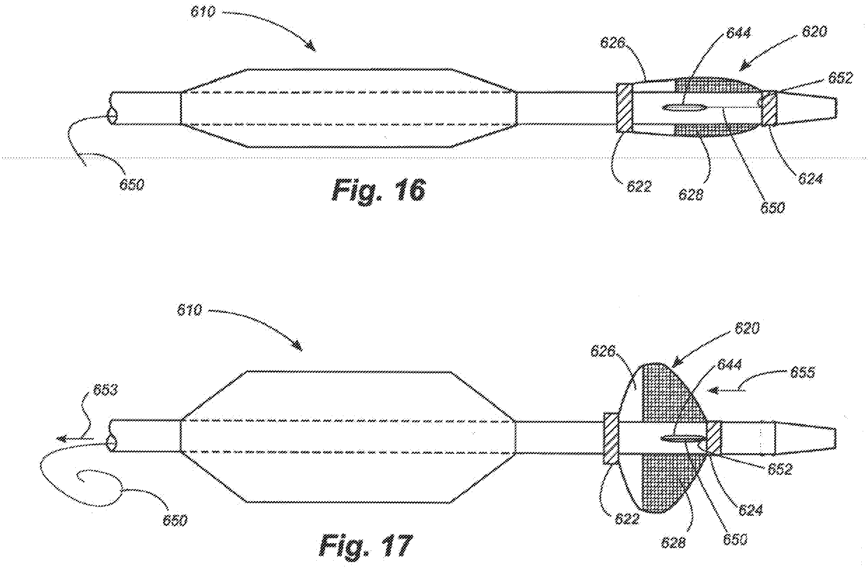

The device **610** shown in Figure **16** and **17** employs a different means for deploying the filter **620.** In the aspect **610** a pull wire **650** can be used. The pull wire **650** can extend through what would formerly have been used as the filter balloon inflation lumen **644,** and the distal end **652** of the pull wire **650** can be attached to the distal ring **624.** When the physician wishes to deploy the filter **620,** he exerts a tension on the wire **650,** as indicated by the arrow **653,** thus drawing the distal ring **624** in a proximal direction as indicated by the arrow **655** toward the proximal ring **622.** The ribs bow **626** outward, deploying the filter mesh **628** as shown in Figure **17****.**

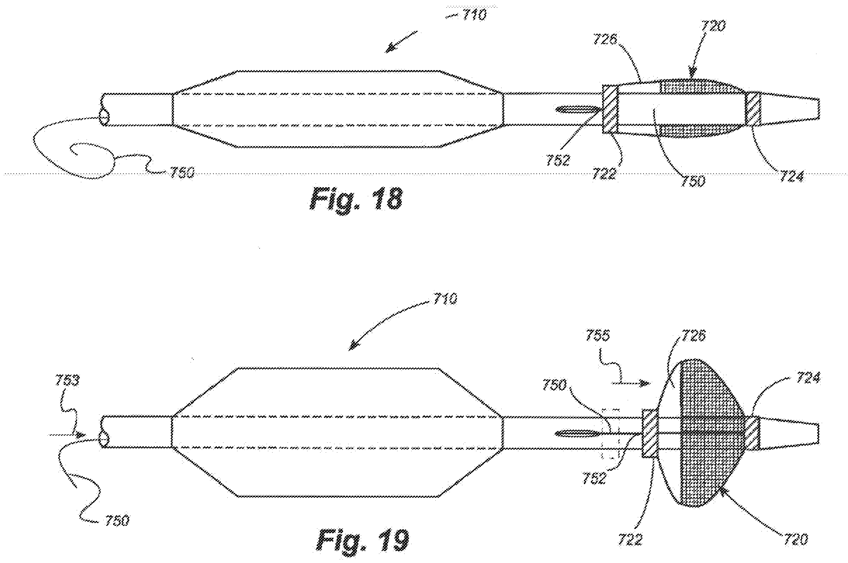

In the device **710** shown in Figures **18** and **19****,** the distal end **752** of a push wire **750** can be attached to the proximal ring **722.** Thus when the wire **750** can be pushed in the direction indicated by the arrow **753,** the proximal ring **722** can be advanced distally toward the distal ring **724** in the direction indicated by the arrow **755,** causing the ribs **726** to bow outward and thereby deploying the filter **720,** as shown in Figure **19****.** Optionally, the disclosed embolic filter can be formed from a shape memory material in which the base or unstrained shape memory is in a baseline open position or a baseline closed position.

When the embolic filter has a baseline open configuration, the wire can be configured to attach to a portion of the proximal movable collar. It will be appreciated that the coupled wire will be held in tension when the catheter is inserted into the body to provide the desired degree of minimal cross-sectional area. Subsequently, when the embolic filter is positioned in the desired location within the patient's blood vessels, the tension can be selectively reduced or released, which will allow for the expansion or opening of the embolic filter as the shape memory material urges or biases the embolic filer to its baseline open position. It is contemplated that once the surgical procedure is complete, i.e., when an exemplary angioplasty procedure has been performed, the wire can be placed under tension and retracted, which pulls the proximal collar proximally and thereby closes the embolic filter.

In a similar aspect, where the embolic filter has a baseline closed position, it will be appreciated that the coupled wire will not need be held in tension when the catheter is inserted into the body to provide the desired degree of minimal cross-sectional area. When the embolic filter is positioned in the desired location within the patient's blood vessels, the wire is placed in compression to advance the activation wire is a forward or distal direction to effect the desired opening of the filter, which acts against the bias force that otherwise urges the embolic filter to the baseline closed position. In this aspect, it is contemplated that once the surgical procedure is complete, the compression force being externally applied to the wire can be released, which allows the shape memory of embolic filter to urge or bias the embolic filer to its baseline closed position and thereby close the embolic filter.

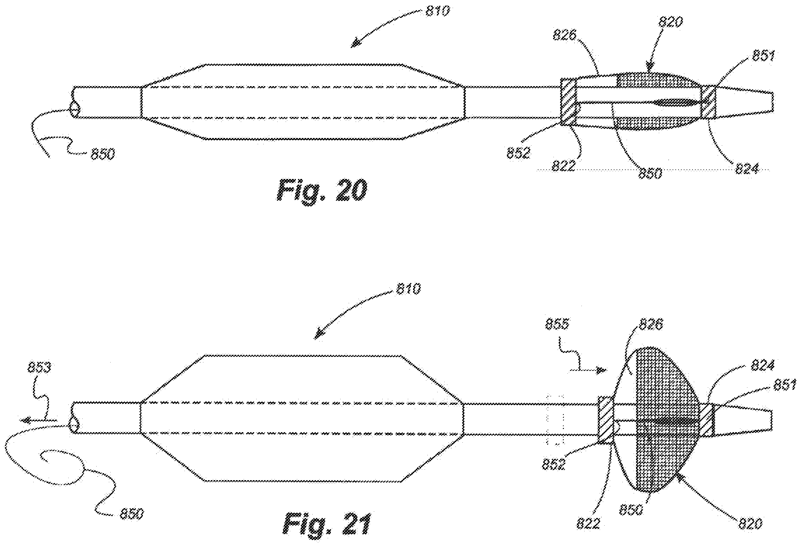

The device **810** shown in Figures **20** and **21** uses a pull wire **850** to erect the filter **820.** The pull wire **850** can wrap around an opening **851** in the stationary distal ring **824** and can extend rearward toward the proximal ring **822** to which the distal end **852** of the pull wire can be attached. Thus when tension can be exerted on the pull wire **850** in the direction indicated by the arrow **853,** the proximal ring **822** can be drawn distally toward the distal ring **824** in the direction indicated by the arrow **855,** causing the ribs **826** to bow outward and thereby deploying the filter **820,** as shown in Figure **21****.**

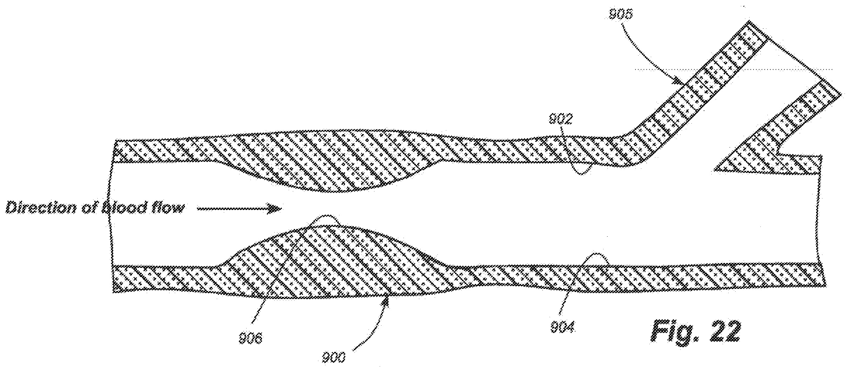

The operation of the device **10** will now be explained with respect to Figures **22-25****,** and it should be understood that the other devices operate on a substantially the same principles. Figure **22** shows a vascular structure (e.g., coronary artery, saphenous vein graft, renal artery, carotid artery, superficial femoral artery, etc.) **900** with upper and lower walls **902, 904,** a branch vessel **905,** and a stenosis or blockage **906** caused by the build-up of plaque or other substances on the arterial walls in such a way as to narrow the diameter of the arterial lumen, and in the process, constrict the flow of blood therethrough.

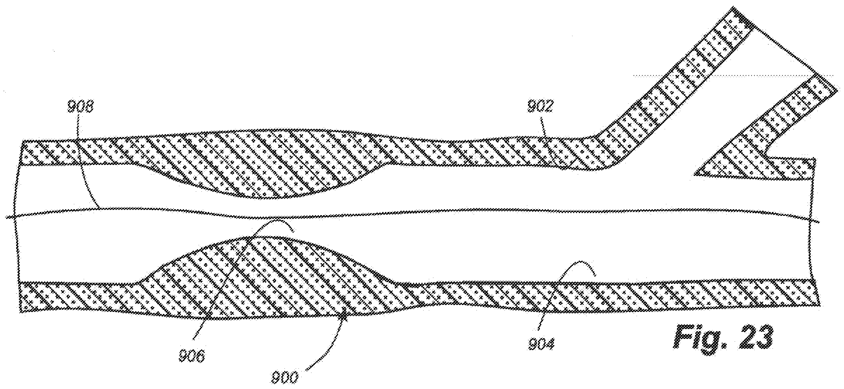

In Figure **23****,** a guide wire **908** has been inserted by the physician, such as through the femoral artery, and guided through the vascular system until the guide wire passes through the stenosis **906** in the vascular structure **900.**

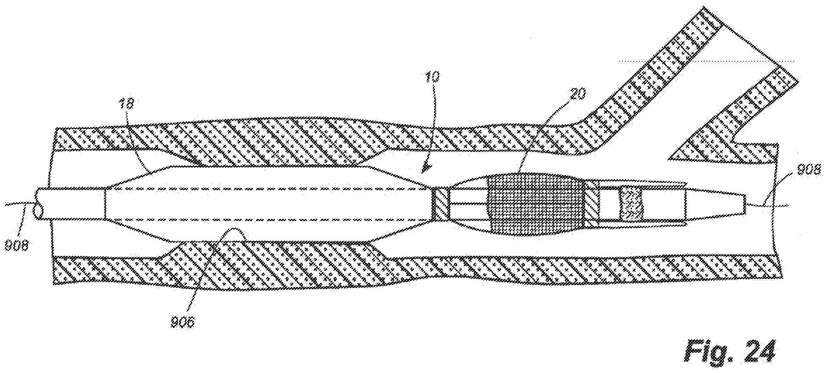

Referring now to Figure **24****,** the apparatus **10** has been inserted over the guide wire 908 and advanced to a location wherein the angioplasty balloon resides within the stenosis **906.** The embolic filter **20** resides a few centimeters distal or downstream from the angioplasty location. In Figure **24** both the angioplasty balloon and the embolic filter can be shown in their un-deployed configurations.

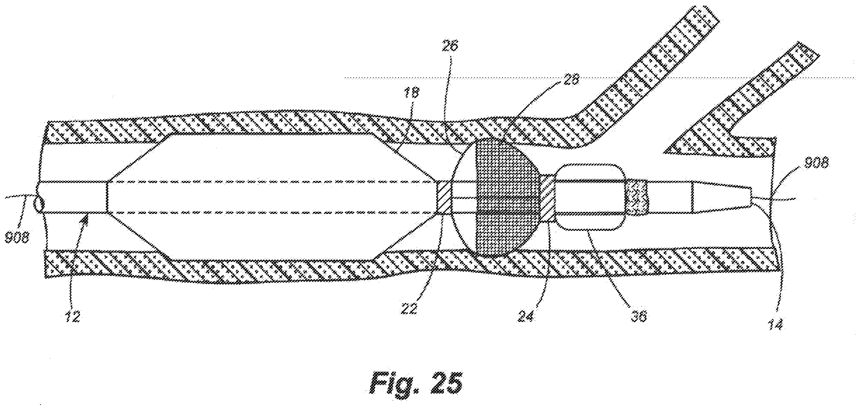

In Figure **25** the embolic filter **20** has been deployed by inflating the filter balloon **36,** causing the distal ring **22** to slide in a proximal direction along the catheter shaft **12.** As the ribs **26** bow outward, the mesh filter material **28** supported by the ribs spreads so as to cover substantially the entire arterial lumen. The angioplasty balloon **18** can be deployed next. As the balloon **18** inflates, it pushes tissue and plaque forming the stenosis **906** outward, opening the stenosis and potentially loosening embolic particles in the process. Any such embolic particles which get released into the blood stream will be caught by the embolic filter **20** and will thereby be prevented from traveling to a location where they can cause injury to the patient.

Figure **25** illustrates the close proximity in which the filter **20** can be deployed relative to the stenosis **906.** Despite the short "landing area", defined as the area between the stenosis **906** and the branch vessel **905,** the filter **20** can be deployed to capture embolic particles upstream of the branch vessel.

When removing the device **10** from the coronary artery, the preferred procedure can be to deflate the angioplasty balloon **18** first, prior to collapsing the embolic filter **20.** In this way, any embolic particles that break loose as the angioplasty balloon **18** deflates can be captured by the filter **20.** The embolic filter balloon **20** can then be deflated, permitting the ribs **26** and filter mesh **28** to collapse against the shaft **14** of the catheter **12.** Any embolic particles captured by the mesh **28** can be trapped against the shaft **14.** The device **10** can be then withdrawn over the guide wire **908** and removed from the patient's body.

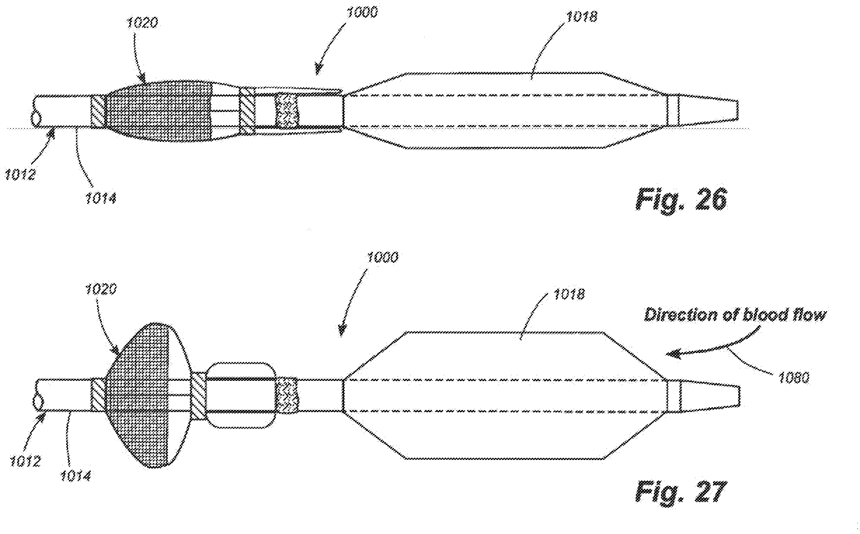

In various peripheral vascular applications, it may be necessary to insert the catheter against the direction of blood flow (e.g., the aorta). Figures **26** and **27** illustrate a device **1000** in which the angioplasty balloon **1018** and the embolic filter **1020** can be reversed on the shaft **1014** of the catheter **1012.** Thus with the blood flowing within the vessel in the direction indicated by the arrow **1080,** the embolic filter **1020** can be proximal to the angioplasty balloon **1018** and thus positioned to capture any embolic particles that may be dislodged by the angioplasty balloon.

While the aspect **1000** of Figures **26** and **27** employs the same method and device for deploying the embolic filter as the aspect **10** of FIGS. **1-3****,** it can be understood that the methods and devices for deploying the embolic filter of other aspects disclosed above can be equally applicable to a configuration like the device of aspect **1000** where the angioplasty balloon can be positioned between the embolic filter and the tip of the device.

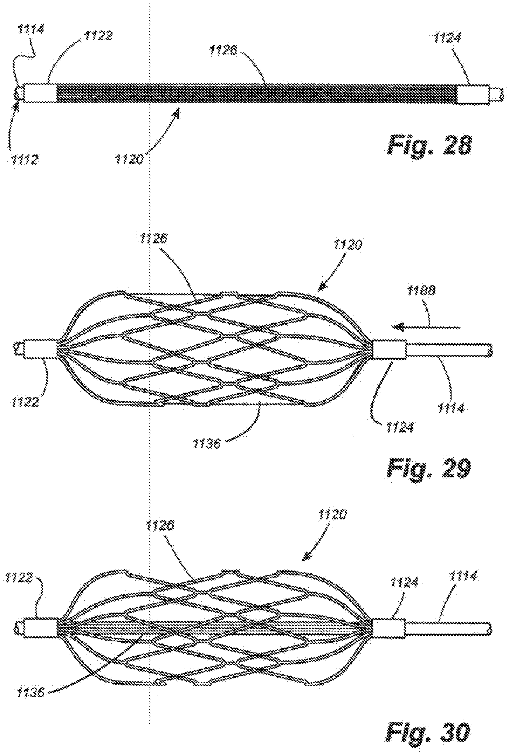

Figures **28-32** show still another embolic filter **1120** for use in conjunction with an angioplasty balloon. FIGS. **28-32** show only the embolic filter **1120** and not the angioplasty balloon, but it can be understood that the embolic filter can be located on the same catheter **1114** as the angioplasty balloon in the same manner as the aspects previously disclosed. Further, FIGS. **29-31** show the embolic filter **1120** without its filter mesh **1128** for clarity of illustration.

In Figure **28** the embolic filter **1120** can be folded closely against the shaft **1114** of the catheter **1112.** The ribs **1126** of the filter **1120** extend between a proximal ring portion **1122** and a distal ring portion **1124.** The distal ring portion **1124** can be slideably mounted on the shaft **1114** of the catheter **1112** and the proximal ring portion **1122** can be fixed with relation to the shaft of the catheter. In Figure **29** the embolic filter balloon **1136** has been inflated, expanding the ribs **1126** of the embolic filter. As the ribs expand, the distal ring portion **1124** slides in the proximal direction, as shown by the arrow **1188.** Once expanded, the ribs **1126** maintain their shape, such that when the embolic filter balloon **1136** deflates as shown in Figure **30****,** the embolic filter **1120** remains expanded.

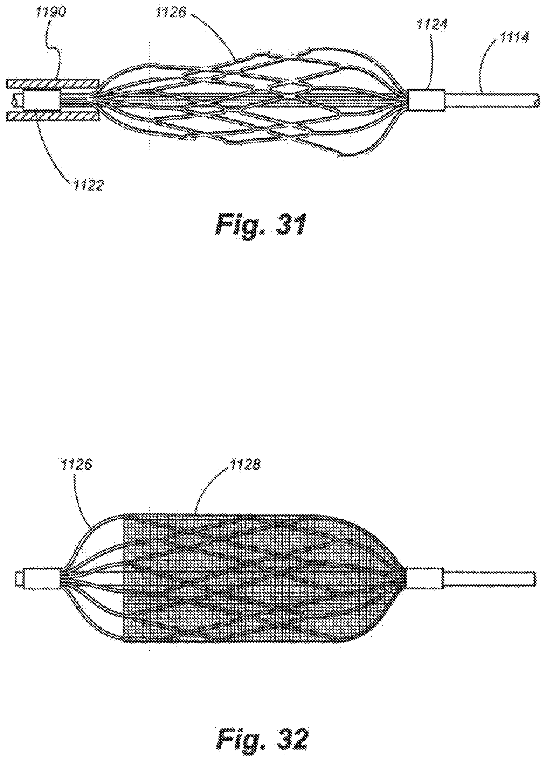

To retract the embolic filter **1120,** a second, outer catheter **1190** can be advanced over the catheter **1112,** as shown in Figure **31****,** causing the ribs **1126** to collapse as the embolic filter can be withdrawn into the forward end of the outer catheter **1190.** As the ribs **1126** collapse, the distal ring portion **1124** slides in the distal direction. Once the embolic filter **1120** has been completely retracted into the forward end of the outer catheter **1190,** the outer and inner catheters can be withdrawn simultaneously.

Figure **32** shows the embolic filter **1120** with filter mesh **1128** positioned over the ribs **1126.**

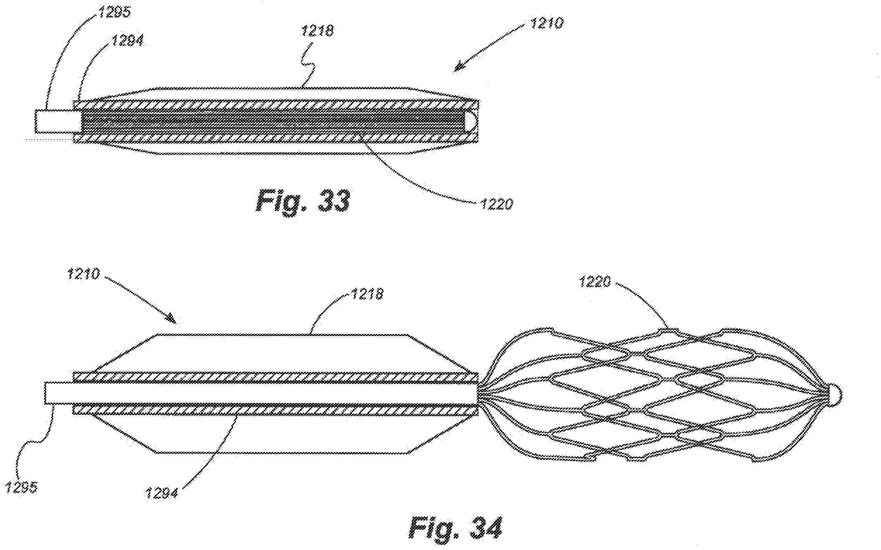

Figures **33** and **34** illustrate a further percutaneous angioplasty device **1210,** in which the embolic filter **1220** can be located on a different carrier than the angioplasty balloon **1218.** Specifically, the angioplasty balloon **1218** can be located on an outer catheter **1294,** and the embolic filter **1220** can be located at the forward end of an inner catheter **1295.** (The embolic filter **1220** is shown without filter mesh in Figures **33** and **34** for clarity of illustration.) The outer catheter preferably has three lumens, one for inflating the angioplasty balloon **1218,** one for accommodating a guide wire (not shown), and one for receiving the inner catheter **1295** and embolic filter **1220.** The inner catheter **1295** can be slideably and telescopically disposed within the outer catheter **1294.** The ribs **1226** of the embolic filter **1220** can be formed from a shape-memory metal such as nitinol and can be constructed to normally assume an "open" configuration. When retracted within the forward end of the outer catheter **1294,** the ribs **1226** of the embolic filter collapse.

To use the percutaneous angioplasty device **1210,** the inner catheter can be inserted into the outer catheter so that the embolic filter **1220** lies within the distal end of the device, as shown in Figure **33****.** The outer and inner catheters **1294, 1295** can be inserted together, such as through the femoral artery, over a guidewire and advanced through the vascular system to a location wherein the deflated angioplasty balloon **1218** resides within the stenosis. Once location of the angioplasty balloon **1218** within the stenosis has been verified by suitable medical imaging technology, the inner catheter can be advanced to move the embolic filter **1220** beyond the forward end of the outer catheter **1294.** As the embolic filter **1220** exits the confines of the outer catheter **1294,** the ribs can assume their expanded configuration and deploy the embolic filter. Thereafter the angioplasty balloon **1218** may be deployed to treat the stenosis, and any emboli loosened during the procedure can be captured by the embolic filter **1220** downstream of the stenosis.

When the angioplasty procedure has been completed, the angioplasty balloon **1218** can be deflated, and the embolic filter **1220** can be withdrawn back into the forward end of the outer catheter **1294,** collapsing the filter. The outer and inner catheters **1294,1295** can be then withdrawn together from the patient.

In the foregoing aspect a wire can be substituted for the inner catheter **1295** as a means for carrying the embolic filter **1220.**

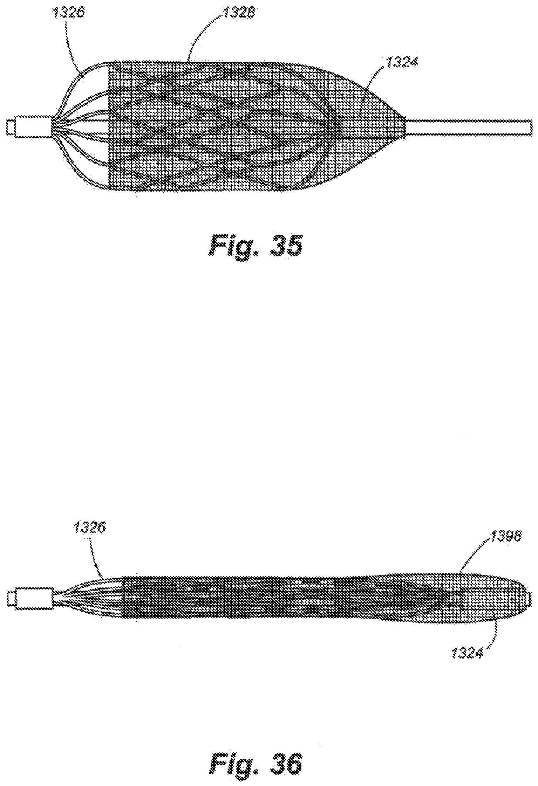

Figures **35** and **36** show an angioplasty device **1310** that can be identical to the device **10,** with the exception that the filter mesh **1328** extends distally beyond the end of the ribs **1326** and can be attached to the distal end of the distal ring **1324.** When the filter **1320** is un-deployed, as shown in Figure **36****,** a sac **1398** can be formed which helps contain the embolic particles, thereby minimizing the possibility that the ribs **1326** will squeeze the particles out of the filter.

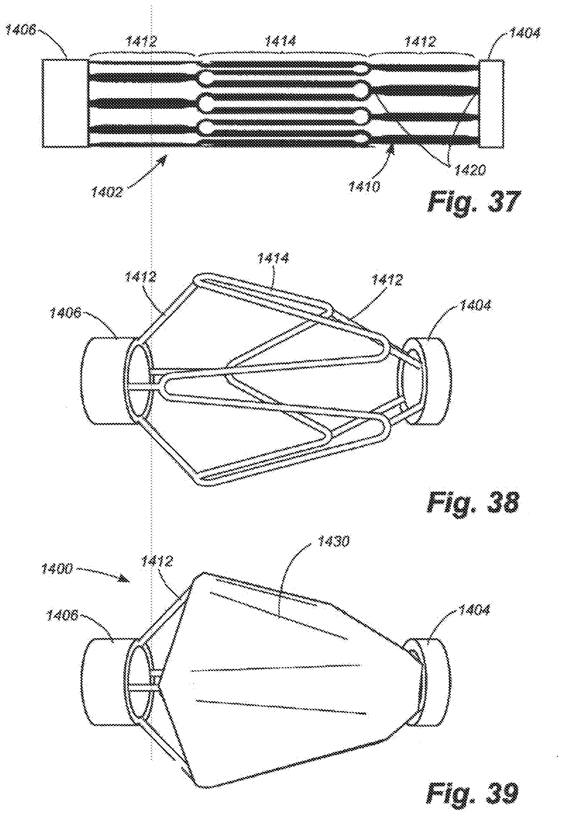

Referring now to Figures **37-39****,** an alternate aspect of a filter **1400** is illustrated. Figure **37** can be a projection of a cylinder, i.e., a generally cylindrical filter frame **1402** can be shown unrolled and flattened. The frame **1402** can be made of flexible material such as Nitinol. The support frame **1402** can comprise a generally tubular shape with a proximal ring **1404** at one end and a distal ring **1406** at the opposite end. Struts **1410** can be attached between the end rings **1404, 1406.** In some of the figures the struts are shown in solid black to facilitate differentiation between the struts and the spaces there between.

More specifically, the struts **1410** of the frame **1402** comprise a plurality of longitudinal struts **1412** at each end of the frame and a connecting plurality of intermediate struts **1414.** The intermediate struts **1414** form a serpentine-like pattern. Points of weakness **1420** can be formed on the struts **1410** in strategic locations to facilitate controlled bending of the frame **1402.** In the present aspect, these points of weakness comprise points of reduced cross-sectional area. Further, in the present aspect these points of weakness can be formed at the connection points between the rings and the longitudinal struts and the connection between the longitudinal struts and the struts of the serpentine pattern. Because of the narrow width at the connection points the longitudinal struts can flare open in the radial direction, while simultaneously causing the serpentine struts to expand radially.

When the proximal and distal rings **1404, 1406** move toward one another, such as by any of the mechanisms hereinabove described, the filter frame **1402** can assume a deployed configuration as shown in Figure **38****.** The longitudinal struts can pivot radially outward, while the serpentine struts can spread apart to permit circumferential expansion.

Figure **39** shows the filter frame **1402** covered in a filter membrane **1430.** The distal end of the filter membrane can be open to permit embolic particles to enter the filter, where they can be trapped by the filter membrane.

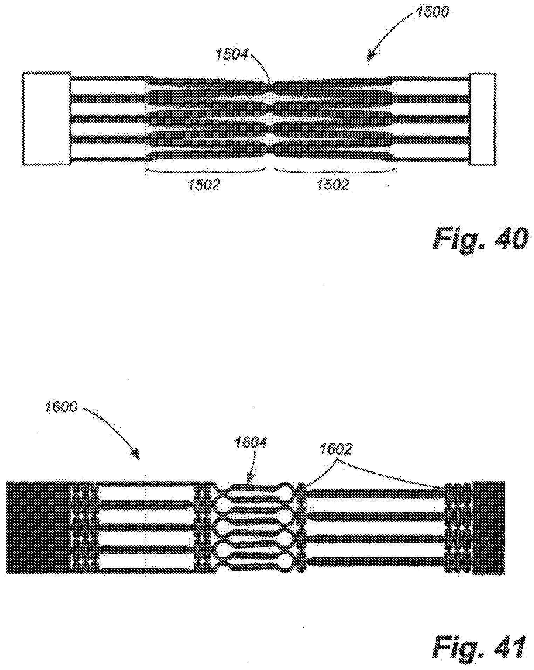

Figures **40** and **41** can be cylindrical projections depicting alternate frame designs. In Figure **40****,** the frame **1500** can comprise two sets of intermediate struts **1502** that can form serpentine patterns. The two sets of intermediate struts **1502** can be joined by connecting members **1504.** Points of weakness can be formed at strategic locations, e.g. at connections between longitudinal and intermediate struts and at the connections between the intermediate struts and the connecting members **1504.**

Figure **41** depicts another aspect of a frame **1600** in which the points of weakness can be formed by circular or oval cutouts **1602** transverse to the longitudinal axis of the struts **1604.** These type of structures **1602** can provide flexibility resulting in easier opening and closing of the frame 1600. These structures **1604** can also reduce the stress induced permanent set and hence, allow the frame **1600** to retract back to its original shape. The oval and/or circular structures **1602** can also provide enough longitudinal rigidity which can urge the filter frame to open.

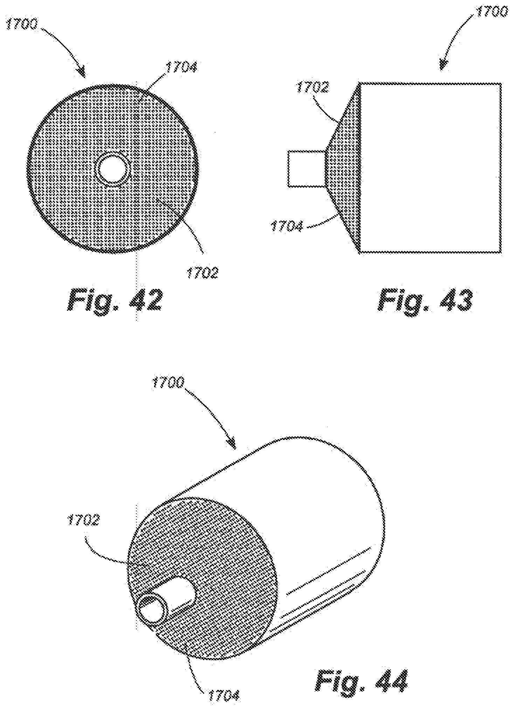

Figures **42-44** illustrate an aspect of a filter membrane **1700.** The filter membrane **1700** can be formed in the shape of a funnel. The conical surface **1702** of the funnel can have a plurality of holes **1704** formed therein. The filter membrane **1700** can comprise semi-compliant material such as nylon or PebaxT or can comprise elastic materials such as thermoplastic elastomers or thermoset elastomers. In an alternative aspect, the filter membrane can comprise a wire mesh filter which can be formed from a wire mesh comprising a plurality of woven metallic or polymeric wires. Some examples of thermoset elastomers polyurethane and copolymers include, but are not limited to. Pellathane™, Tecothane™, Chronofles™, and the like. These materials can allow placement of the holes **1704** close to each other. In one exemplary embodiment, the size of the holes **1704** can be about 40 microns, and the holes **1704** can be placed about 40 microns apart.

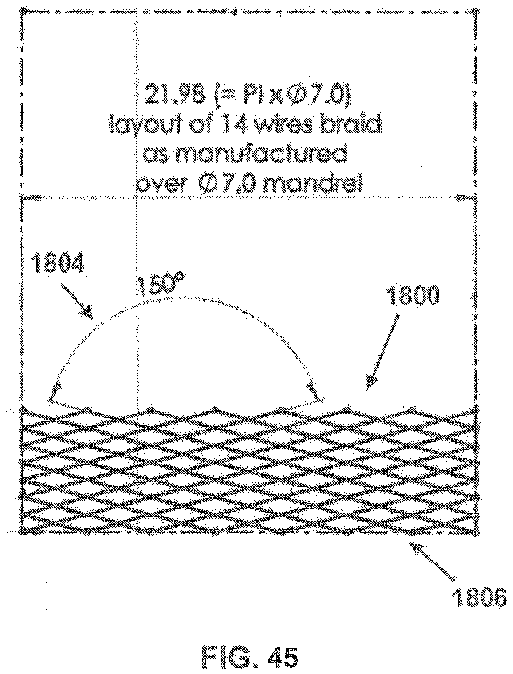

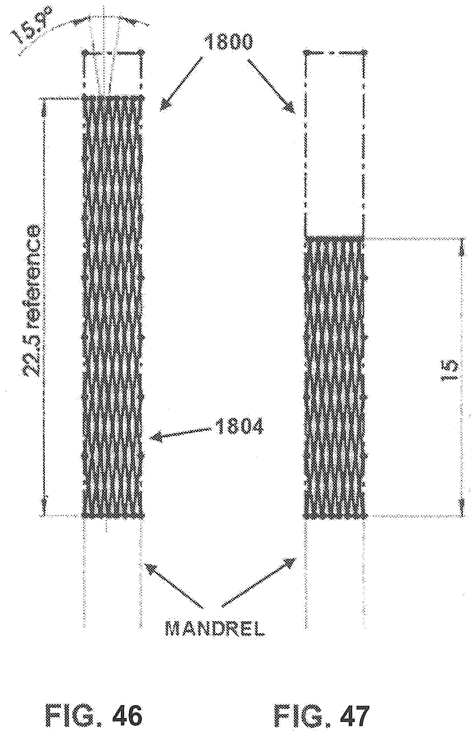

Referring now to Figures **45**-**48**, the frame formed from braided wires to form a wire mesh frame **1800** is illustrated. Figure **45** shows a perspective view of an alternate frame design shown unrolled and flattened. In this aspect, it will be appreciated that the wire mesh frame **1800** has a generally cylindrical shape when formed. Figure **46** shows the unformed wire mesh being stretched out over a mandrel to form a substantially cylindrical shape and Figure **47** shows the respective ends of the formed frame design being trimmed to form a desired elongate longitudinal length. In various optional aspects, it is contemplated that the resultant trimmed cylindrically shaped frame design can be heat treated to set the shape memory in the formed position or, optionally, the trimmed frame can be compressed to a desired dimension and then heat treated to set the shape memory in the formed position (baseline closed position) or it can be expanded to a desired dimension and then heat treated to set the shape memory in the formed position (baseline open position).

In this aspect, one skilled in the art can appreciate that wire braiding techniques can be employed to form the wire mesh frame **1800** that are similar to those used to manufacture stents, closure devices, intra-vascular devices and the like. The wires can comprise, for example and without limitation, metal wires, polymer wires and the like. In one aspect, the frame can be formed from a wire braid comprising from about 12 to about 16 wires. In another aspect, the wire mesh frame un-deployed diameter can be from about 0.8 to about 1.0 mm and can be adapted to slidingly fit the catheter shaft diameter. The lead angle between the wires comprising the wire mesh from can be selected to be relatively low to allow the wire mesh frame **1800** to open to a relatively high diameter when deployed. This deployed diameter **1806** can be about 4-7 mm. The wires comprising the wire mesh frame can have a rounded profile in cross-section. The wires comprising the wire mesh frame can also be from about 0.0508 mm (0.002") to about 0.0762 mm (0.003") in diameter or, alternatively the wires can be flat. If the wires are selected to be flat, the wire can be further configured to be about 0.0254 mm x 0.0762 mm (0.001" x 0.003") in cross-section in order to reduce the profile of the wire mesh frame.

In one embodiment, the braided wire mesh frame 1800 can be formed from 14 Nitinol or Cobalt-Chromium round wires having a 60 micron diameter and a braiding angle 1804 of about 150 degrees on a 7 mm shaft that corresponds to the maximum deployed diameter. In this aspect, the braiding angle can be defined as double (2X) the angle between the wire and the central axis. Optionally, it is contemplated that the braiding angle can be between about 1.5X and 4X or be between 1.7X and 3X. In this aspect, it is contemplated that the braided wire mesh frame **1800** can then be compressed to un-deployed diameter of about a 1 mm and heat treated to shape set the form, i.e., to set the base or unstrained shape memory in a base line closed position. Of course, it is also contemplated that the braided wire mesh frame **1800** can be heat treated to set the base or unstrained shape memory in a base line open position. It is contemplated that the wire mesh frame can form a relatively wide mesh when opened in order to allow blood flow into the filter membrane. It is also contemplated that the wire mesh frame can comprise less than 12 wires or more than 16 wires, depending on the desired inhibition or lack thereof to the flow of blood.

As shown in Figure **16****,** a wire mesh frame **1800** can be incorporated into the device by joining the distal end **1808** of the wire mesh frame to a distal ring. In one aspect, the distal end of the wire mesh frame can be thermally bonded to a polymer or metallic distal ring. The distal ring can be adapted to slideably fit over the catheter shaft. The proximal end **1810** of the wire mesh frame can be attached to the proximal ring by thermal bonding as described above or other methods known to one skilled in the art. The distal and proximal rings can be of any length and diameter, but in one aspect, both the proximal and distal ends can have a length from about 0.5 to about 2.0 mm.

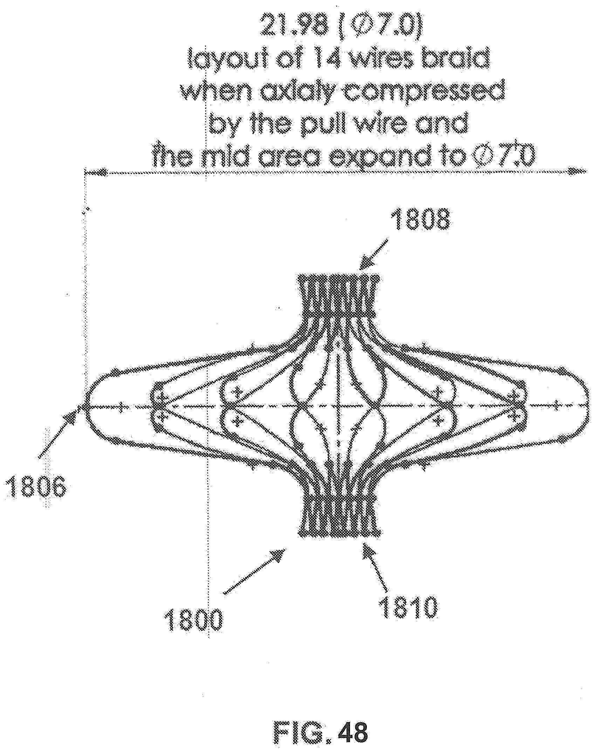

In one exemplary aspect, Figure **48** shows an exemplary trimmed cylindrically shaped frame design that has been heat treated to set the shape memory in the baseline closed position upon application of an axial compression. For example and not meant to be limiting, the applied axial compression can be exerted by an external operator pulling on a pull wire to controllably cause the mid-portion of the trimmed cylindrically shaped frame design to expand to a desired diameter. This desired diameter is a multiple of the original diameter of the frame design in the baseline closed position.

The filter membrane **1700** and **1800** can be attached to a support frame, such as the frames **1400, 1500,** or **1600** hereinabove described, such that it covers one end of the frame as well as the centrally located serpentine strut structure. The set of longitudinal struts of the filter frame can remain exposed. The filter membrane **1700** can be attached on the outside of the frame or on the inside of the frame. In addition, it is contemplated that the distal end of the membrane can be terminated at the distal ring or can extend beyond the ring to attach to the shaft of the catheter distal to the distal ring.

In the wire mesh frame **1800,** the filter membrane can be attached to the wires comprising the frame **1800** at a location that is approximately equidistant between the proximal and distal ends of the wire mesh frame as it is positioned on the catheter shaft. In a further aspect, the configuration of the device depicted in Figure **16** and described in the corresponding text can be employed with the wire mesh frame **1800.** Here, a pull wire operable from outside the patient which can be attached to a distal ring of the embolic filter. When the physician exerts tension on the wire, the distal ring can be displaced proximally, bringing it closer to the proximal ring, causing the ribs to bow outward and thereby deploying the embolic mesh filter. The wire mesh frame **1800** can expand to urge the membrane edge into contact with the blood vessel wall, thus directing blood to flow through the membrane to filter any embolic particles from the blood flow. Subsequent to the procedure, the pull wire can be advanced allowing the wire mesh frame to compress back to its un-deployed state to facilitate removal of the device from the patient.

In light of the foregoing disclosure, one skilled in the art will be able to appreciate the advantages of a braided wire mesh frame **1800** relative to a laser-cut frame. The braided wire mesh frame can be more flexible, increasing the ease of navigation through tortuous anatomies to the target site. The braided wire mesh frame can also seat more tightly on the catheter shaft in an un-deployed state and lack the struts or any other projections that could potentially disengage the frame from catheter shaft during delivery or withdrawal of the device through tortuous anatomy or through previously deployed stents or other vascular devices. A braided frame can expand to create a rounded sealing edge together with the membrane edge regardless of whether it's expanded in straight or curved vessel location and creates a greater number of compression points to seal the membrane to the vessel wall around the circumference of the vessel. Further, the braided wire mesh frame more easily compresses to a smaller diameter after expansion, increasing the ease of withdrawal.

The filters herein depicted can be deployed by pulling or pushing an actuation wire or inflating an actuation balloon, depending on the type of catheter chassis being used. As the filter deploys, the serpentine struts expand circumferentially. The filter membrane is thus deployed. Upon removal of the actuation force, the filter can retract to its normally closed position.

An advantage of the filter material can be that its natural shape can be in a closed or un-deployed condition. The filter material can stretch as the filter deploys and collapse to its normal condition when the frame retracts. Therefore, the membrane has no permanent set during storage and can always be expanded to a correct size. Further, because the filter collapses under the resiliency of the filter material, the filter does not require a recovery sheath. If needed, however, a sheath may be used to further collapse the filter containing embolic debris prior to retrieval.

In an optional aspect, the filters of the disclosed aspect can be characterized by a long filter body that opposes the vessel wall over a greater area, thus reducing the chance of leakage between the filter and the vessel wall.

In each of the foregoing examples, it will be appreciated that an angioplasty balloon can be but one means for relieving a stenosis in a vessel. Stents, mechanical thrombectomy devices, or other suitable apparatus may be substituted for the angioplasty balloon and positioned on the catheter at a location proximal to the embolic filter. Thus any emboli loosened by the stent or mechanical thrombectomy device can be captured by the embolic filter in the same manner as described above with respect to the angioplasty balloon.

While the foregoing disclosed aspects comprise filter ribs of a shape memory metal such as nitinol, it can be appreciated that similar results can be obtained by using any suitable resilient material. The ribs would be formed straight, forced open by the balloon, and return to their normal shape as a result of the resiliency of the structure. Or, in the case of the aspect of Figures **33** and **34**, the ribs would be initially formed in an open position, deformed inwardly to fit within the outer catheter, and return to their normal open position when released from the confines of the outer catheter.

Variations in the design of the filter can be also contemplated. For example, while both ends of the ribs **26** of the filter **20** can be mounted to rings **22, 24,** it can be appreciated that the ends of the ribs at the fixed end of the filter can be secured directly to the catheter shaft.

Thus, implementations of the foregoing provide various desirable features. For instance, the present disclosure permits the placement of the embolic filter very close to the means for treating the stenosis. This has the effect of minimizing the "landing area" of the filter and also permits the protection of side branches, as shown in Figures **22-25****.**

The described aspects are to be considered in all respects only as illustrative and not restrictive. The scope of the invention is, therefore, indicated by the appended claims rather than by the foregoing description. All changes that come within the meaning and range of equivalency of the claims are to be embraced within their scope.

## Claims

1. A percutaneous transluminal angioplasty device (610, 710, 810), comprising:
an elongated catheter (12) having proximal and distal (16) ends;
an interventional device (18) attached to the catheter (12) adjacent the distal end (16) thereof;
a filter (620, 720, 820) attached to the elongated catheter (12) between the interventional device (18) and the distal end (16) of the catheter (12), the filter (620, 720, 820) being collapsible for insertion and removal of the distal end (16) of the catheter (12) into a blood vessel (900), and the filter (620, 720, 820) being expandable to an expanded position to capture emboli released into a bloodstream by operation of the interventional device (18), wherein the filter (620, 720, 820) comprises:
a movable ring portion (624, 722, 822) movably attached to the catheter (12);
a fixed ring portion (622, 724, 824) immovably attached to the catheter (12) such that the movable ring portion is movable relative to the fixed ring portion;
wherein the percutaneous transluminal angioplasty device (610, 710, 810) is **characterized by** further comprising a braided wire mesh frame (1800) that is formed of a shape memory material that urges the filter (620, 720, 820) into a base line closed or collapsed position, a distal end (1808) of the braided wire mesh frame (1800) is coupled to the movable ring portion (624, 722, 822) and a proximal end (1810) of the braided wire mesh frame (1800) is coupled to the fixed ring portion (622, 724, 824); and
a filter membrane (1700) overlying a portion of the braided wire mesh frame (1800);
wherein the braided wire mesh frame (1800) is flexible and configured to seal the filter membrane (1700) to the vessel wall (902, 904) around the circumference of the vessel (900) in a straight or a curved vessel location; and wherein, in the expanded position, the braided wire mesh frame (1800) urges the membrane into contact with the blood vessel wall (902, 904); and
wherein the catheter (12) further comprises a lumen (44, 644) extending from a location proximate the proximal end of the catheter (12), to a location distal to the interventional device (18); and
an actuator wire (650, 750, 850) having proximal and distal (652, 752, 852) ends, the actuator wire (650, 750, 850) extending through the lumen (44, 644) of the catheter (12), the proximal end of the actuator wire (650, 750, 850) extending to a location proximate the proximal end of the catheter (12) and the distal end (652, 752, 852) of the actuator wire (650, 750, 850) exiting the lumen (44, 644) through the side wall of the catheter (12) at the location distal to the interventional device (18), the distal end (652, 752, 852) of the actuator wire (650, 750, 850) being attached to the movable ring portion (624, 722, 822); wherein when the filter (620, 720, 820) is in a collapsed condition, manipulating the proximal end of the wire (650, 750, 850) exerts a force on the movable ring portion (624, 722, 822) that moves the movable ring portion (624, 722, 822) toward the fixed ring portion (622, 724, 824) and causes the braided wire mesh frame (1800) to bow outward to the expanded position.

2. The percutaneous transluminal angioplasty device (610, 710, 810) of Claim 1, wherein the movable ring portion is the distal ring portion (624, 724, 824).

3. The percutaneous transluminal angioplasty device (610, 710, 810) of Claim 1, wherein the distal end (652, 752, 852) of the actuator wire (650, 750, 850) exits the lumen (44, 644) through the catheter (12) side wall at a location distal to the proximal ring portion (622, 722, 822).

4. The percutaneous transluminal angioplasty device (610, 710, 810) of Claim 3, wherein the distal end (652, 752, 852) of the actuator wire (650, 750, 850) is operatively connected to the distal ring portion (624, 724, 824).

5. The percutaneous transluminal angioplasty device (610, 710, 810) of Claim 4, wherein pulling on the proximal end of the actuator wire (650, 750, 850) draws the distal ring portion (624, 724, 824) toward the fixed proximal ring portion (622, 722, 822).

6. The percutaneous transluminal angioplasty device (610, 710, 810) of Claim 1, wherein the interventional device (18) comprises an angioplasty balloon (18).

7. The percutaneous transluminal angioplasty device (610, 710, 810) of Claim 1, wherein the interventional device (18) comprises a stent.

8. The percutaneous transluminal angioplasty device (610, 710, 810) of Claim 1, wherein the interventional device (18) comprises a mechanical thrombectomy device.

9. The percutaneous transluminal angioplasty device (610, 710, 810) of Claim 1, wherein the shape memory material comprises Nitinol or Cobalt-Chromium.

10. The percutaneous transluminal angioplasty device of (610, 710, 810) Claim 1, wherein filter membrane (1700) overlies a distal portion (1808) of the braided wire mesh frame (1800).

11. The percutaneous transluminal angioplasty device (610, 710, 810) of Claim 1, wherein the filter membrane (1700) extends beyond the braided wire mesh frame (1800) in a longitudinal direction relative to the longitudinal axis of the catheter (12), such that a sac (1398) is formed to retain embolic particles when the filter (620, 720, 820) is in the collapsed position.

12. The percutaneous transluminal angioplasty device (610, 710, 810) of Claim 1, wherein the braided wire mesh frame (1800) comprises metal wires, polymer wires and the like.

13. The percutaneous transluminal angioplasty device (610, 710, 810) of Claim 1, wherein the braided wire mesh frame (1800) is formed from a wire braid comprising from between about 12 to about 16 wires.

14. The percutaneous transluminal angioplasty device (610, 710, 810) of Claim 1, wherein a braiding angle (1804) between the wires of the braided wire mesh frame (1800) and a longitudinal axis of the braided wire mesh frame (1800) is a multiple between about 1.5X and 4X of the angle between the wire and the central axis when the wire is in the base line closed or collapsed position.

15. The percutaneous transluminal angioplasty device of (610, 710, 810) any one of claims 1 to 14, wherein the braided wire mesh frame (1800) is formed from round wires having a 60 micron diameter and a braiding angle, on a 7 mm shaft, that corresponds to the maximum deployed diameter (1806) which is double (2X) the angle between the wire and the central axis, wherein optionally the braiding angle is 150 °.

16. The percutaneous transluminal angioplasty device (610, 710, 810) of any one of claims 1 to 15, wherein the braided wire mesh frame (1800) forms a wide mesh when opened to allow blood flow into the filter membrane (1700).

## Patentansprüche

1. Perkutane transluminale Angioplastie-Vorrichtung (610, 710, 810), die
einen langgestreckten Katheter (12) mit proximalen und distalen (16) Enden aufweist;
eine interventionelle Vorrichtung (18) aufweist, die an dem Katheter (12) angrenzend an das distale Ende (16) befestigt ist;
einen Filter (620, 720, 820) aufweist, der an dem langgestreckten Katheter (12) zwischen der interventionellen Vorrichtung (18) und dem distalen Ende (16) des Katheters (12) befestigt ist, wobei der Filter (620, 720, 820) zum Einsetzen und Entfernen des distalen Endes (16) des Katheters (12) in ein Blutgefäß (900) zusammenlegbar ist, und der Filter (620, 720, 820) in eine expandierte Position expandierbar ist, um in einen Blutkreislauf freigesetzte Embolien durch den Betrieb der interventionellen Vorrichtung (18) einzufangen, wobei der Filter (620, 720, 820)
einen beweglichen Ringabschnitt (624, 722, 822) aufweist, der beweglich an dem Katheter (12) befestigt ist;
einen festen Ringabschnitt (622, 724, 824) aufweist, der unbeweglich an dem Katheter (12) befestigt ist, so dass der bewegliche Ringabschnitt relativ zu dem festen Ringabschnitt bewegbar ist;
wobei die perkutane transluminale Angioplastie-Vorrichtung (610, 710, 810) **dadurch gekennzeichnet ist, dass** sie ferner einen geflochtenen Drahtgitterrahmen (1800) aufweist, der aus einem Formgedächtnismaterial gebildet ist, das den Filter (620, 720, 820) in eine geschlossene oder eine zusammengelegte Grundlinienposition drängt, wobei ein distales Ende (1808) des geflochtenen Drahtgitterrahmens (1800) an den beweglichen Ringabschnitt (624, 722, 822) gekoppelt ist und ein proximales Ende (1810) des geflochtenen Drahtgitterrahmens (1800) an den festen Ringabschnitt (622, 724, 824) gekoppelt ist; und
eine Filtermembran (1700) aufweist, die über einem Teil des geflochtenen Drahtgitterrahmens (1800) liegt;
wobei der geflochtene Drahtgitterrahmen (1800) flexibel ist und konfiguriert ist, um die Filtermembran (1700) an die Gefäßwand (902, 904) um den Umfang des Gefäßes (900) in einer geraden oder einer gekrümmten Gefäßposition dicht anzudrücken; und wobei, in der expandierten Position, der geflochtene Drahtgitterrahmen (1800) die Membran in Kontakt mit der Blutgefäßwand (902, 904) drängt; und
wobei der Katheter (12) ferner ein Lumen (44, 644) aufweist, das sich von einer Stelle nahe dem proximalen Ende des Katheters (12) zu einer Stelle distal zu der interventionellen Vorrichtung (18) erstreckt; und
einen Aktuatordraht (650, 750, 850) mit proximalen und distalen (652, 752, 852) Enden aufweist, wobei sich der Aktuatordraht (650, 750, 850) durch das Lumen (44, 644) des Katheters (12) erstreckt, wobei sich das proximale Ende des Aktuatordrahts (650, 750, 850) zu einer Stelle nahe dem proximalen Ende des Katheters (12) erstreckt und das distale Ende (652, 752, 852) des Aktuatordrahts (650, 750, 850) das Lumen (44, 644) durch die Seitenwand des Katheters (12) an der Stelle distal zur interventionellen Vorrichtung (18) verlässt, wobei das distale Ende (652, 752, 852) des Aktuatordrahts (650, 750, 850) an dem beweglichen Ringabschnitt (624, 722, 822) befestigt ist; wobei, wenn der Filter (620, 720, 820) in einem zusammengelegten Zustand ist, das Manipulieren des proximalen Endes des Drahtes (650, 750, 850) eine Kraft auf den beweglichen Ringabschnitt (624, 722, 822) ausübt, die den beweglichen Ringabschnitt (624, 722, 822) in Richtung des festen Ringabschnitts (622, 724, 824) bewegt und bewirkt, dass sich der geflochtene Drahtgitterrahmen (1800) nach außen in die expandierte Position biegt.

2. Perkutane transluminale Angioplastie-Vorrichtung (610, 710, 810) nach Anspruch 1, wobei der bewegliche Ringabschnitt der distale Ringabschnitt (624, 724, 824) ist.

3. Perkutane transluminale Angioplastie-Vorrichtung (610, 710, 810) nach Anspruch 1, wobei das distale Ende (652, 752, 852) des Aktuatordrahts (650, 750, 850) das Lumen (44, 644) durch die Katheter(12)-Seitenwand an einer Stelle distal zum proximalen Ringabschnitt (622, 722, 822) verlässt.

4. Perkutane transluminale Angioplastie-Vorrichtung (610, 710, 810) nach Anspruch 3, wobei das distale Ende (652, 752, 852) des Aktuatordrahts (650, 750, 850) operativ mit dem distalen Ringabschnitt (624, 724, 824) verbunden ist.

5. Perkutane transluminale Angioplastie-Vorrichtung (610, 710, 810) nach Anspruch 4, wobei Ziehen am proximalen Ende des Aktuatordrahts (650, 750, 850) den distalen Ringabschnitt (624, 724, 824) in Richtung des festen proximalen Ringabschnitts (622, 722, 822) zieht.

6. Perkutane transluminale Angioplastie-Vorrichtung (610, 710, 810) nach Anspruch 1, wobei die interventionelle Vorrichtung (18) einen Angioplastie-Ballon (18) aufweist.

7. Perkutane transluminale Angioplastie-Vorrichtung (610, 710, 810) nach Anspruch 1, wobei die interventionelle Vorrichtung (18) einen Stent aufweist.

8. Perkutane transluminale Angioplastie-Vorrichtung (610, 710, 810) nach Anspruch 1, wobei die interventionelle Vorrichtung (18) eine mechanische Thrombektomie-Vorrichtung aufweist.

9. Perkutane transluminale Angioplastie-Vorrichtung (610, 710, 810) nach Anspruch 1, wobei das Formgedächtnismaterial Nitinol oder Cobalt-Chrom aufweist.

10. Perkutane transluminale Angioplastie-Vorrichtung (610, 710, 810) nach Anspruch 1, wobei die Filtermembran (1700) über einem distalen Abschnitt (1808) des geflochtenen Drahtgitterrahmens (1800) liegt.

11. Perkutane transluminale Angioplastie-Vorrichtung (610, 710, 810) nach Anspruch 1, wobei sich die Filtermembran (1700) über den geflochtenen Drahtgitterrahmen (1800) hinaus in Längsrichtung zur Längsachse des Katheters (12) erstreckt, so dass ein Sack (1398) gebildet wird, um embolische Teilchen zurückzuhalten, wenn sich der Filter (620, 720, 820) in der zusammengelegten Position befindet.

12. Perkutane transluminale Angioplastie-Vorrichtung (610, 710, 810) nach Anspruch 1, wobei der geflochtene Drahtgitterrahmen (1800) Metalldrähte, Polymerdrähte und dergleichen aufweist.

13. Perkutane transluminale Angioplastie-Vorrichtung (610, 710, 810) nach Anspruch 1, wobei der geflochtene Drahtgitterrahmen (1800) aus einem Drahtgeflecht gebildet ist, das etwa 12 bis etwa 16 Drähte aufweist.

14. Perkutane transluminale Angioplastie-Vorrichtung (610, 710, 810) nach Anspruch 1, wobei ein Flechtwinkel (1804) zwischen den Drähten des geflochtenen Drahtgitterrahmens (1800) und einer Längsachse des geflochtenen Drahtgitterrahmens (1800) ein Vielfaches zwischen etwa 1,5X und 4X des Winkels zwischen dem Draht und der Mittelachse ist, wenn der Draht in der geschlossenen oder zusammengelegten Grundlinienposition ist.

15. Perkutane transluminale Angioplastie-Vorrichtung (610, 710, 810) nach einem der Ansprüche 1 bis 14, wobei der geflochtene Drahtgitterrahmen (1800) aus runden Drähten gebildet ist, die einen Durchmesser von 60 µm und einen Flechtwinkel, auf einer 7 mm-Welle, haben, der dem maximal eingesetzten Durchmesser (1806) entspricht, der das Doppelte (2X) des Winkels zwischen dem Draht und der Mittelachse ist, wobei gegebenenfalls der Flechtwinkel 150° beträgt.

16. Perkutane transluminale Angioplastie-Vorrichtung (610, 710, 810) nach einem der Ansprüche 1 bis 15, wobei der geflochtene Drahtgitterrahmen (1800) ein weites Gitter bildet, wenn er geöffnet ist, um einen Blutfluss in die Filtermembran (1700) zu ermöglichen.

## Revendications

1. Dispositif d'angioplastie transluminale percutanée (610, 710, 810) comprenant :
un cathéter allongé (12) comportant des extrémités proximale et distale (16) ;
un dispositif d'intervention (18) fixé au cathéter (12) de manière adjacente à son extrémité distale (16) ;
un filtre (620, 720, 820) fixé au cathéter allongé (12) entre le dispositif d'intervention (18) et l'extrémité distale (16) du cathéter (12), le filtre (620, 720, 820) étant compressible ou pliable à des fins d'insertion et de retrait de l'extrémité distale (16) du cathéter (12) dans un vaisseau sanguin (900) et hors de celui-ci, et le filtre (620, 720, 820) étant déployable jusqu'à une position déployée afin de capturer des emboles libérés dans une circulation sanguine par la mise en oeuvre du dispositif d'intervention (18), le filtre (620, 720, 820) comprenant :
une partie annulaire mobile (624, 722, 822) fixée de manière mobile au cathéter (12) ;
une partie annulaire fixe (622, 724, 824) fixée de manière immobile au cathéter (12) de telle sorte que la partie annulaire mobile soit mobile par rapport à la partie annulaire fixe ;
le dispositif d'angioplastie transluminale percutanée (610, 710, 810) étant **caractérisé en ce qu'**il comprend en outre une structure à mailles en fils tressés (1800) qui est constituée d'un matériau à mémoire de forme qui sollicite le filtre (620, 720, 820) vers une position de référence fermée ou comprimée, une extrémité distale (1808) de la structure à mailles en fils tressés (1800) étant accouplée à la partie annulaire mobile (624, 722, 822) et une extrémité proximale (1810) de la structure à mailles en fils tressés (1800) étant accouplée à la partie annulaire fixe (622, 724, 824) ; et
une membrane de filtration (1700) recouvrant une partie de la structure à mailles en fils tressés (1800) ;
la structure à mailles en fils tressés (1800) étant souple et configurée pour appliquer la membrane de filtration (1700) à étanchéité sur la paroi du vaisseau (902, 904) autour de la circonférence du vaisseau (900) au niveau d'un emplacement de vaisseau droit ou courbe ; et la structure à mailles en fils tressés (1800), dans la position déployée, sollicitant la membrane de sorte qu'elle vienne en contact avec la paroi du vaisseau sanguin (902, 904) ; et
le cathéter (12) comprenant en outre une lumière (44, 644) s'étendant à partir d'un emplacement situé à proximité de l'extrémité proximale du cathéter (12), jusqu'à un emplacement distal vis-à-vis du dispositif d'intervention (18) ; et
un fil d'actionnement (650, 750, 850) comportant des extrémités proximale et distale (652, 752, 852), le fil d'actionnement (650, 750, 850) s'étendant à travers la lumière (44, 644) du cathéter (12), l'extrémité proximale du fil d'actionnement (650, 750, 850) s'étendant jusqu'à un emplacement situé à proximité de l'extrémité proximale du cathéter (12) et l'extrémité distale (652, 752, 852) du fil d'actionnement (650, 750, 850) sortant de la lumière (44, 644) à travers la paroi latérale du cathéter (12) au niveau de l'emplacement distal vis-à-vis du dispositif d'intervention (18), l'extrémité distale (652, 752, 852) du fil d'actionnement (650, 750, 850) étant fixée à la partie annulaire mobile (624, 722, 822) ; une manipulation de l'extrémité proximale du fil (650, 750, 850), lorsque le filtre (620, 720, 820) se trouve dans un état comprimé, exerçant une force sur la partie annulaire mobile (624, 722, 822) qui déplace la partie annulaire mobile (624, 722, 822) en direction de la partie annulaire fixe (622, 724, 824) et provoquant une flexion de la structure à mailles en fils tressés (1800) vers l'extérieur jusqu'à la position déployée.

2. Dispositif d'angioplastie transluminale percutanée (610, 710, 810) selon la revendication 1, dans lequel la partie annulaire mobile est la partie annulaire distale (624, 724, 824).

3. Dispositif d'angioplastie transluminale percutanée (610, 710, 810) selon la revendication 1, dans lequel l'extrémité distale (652, 752, 852) du fil d'actionnement (650, 750, 850) sort de la lumière (44, 644) à travers la paroi latérale du cathéter (12) au niveau d'un emplacement distal vis-à-vis de la partie annulaire proximale (622, 722, 822).

4. Dispositif d'angioplastie transluminale percutanée (610, 710, 810) selon la revendication 3, dans lequel l'extrémité distale (652, 752, 852) du fil d'actionnement (650, 750, 850) est raccordée de manière fonctionnelle avec la partie annulaire distale (624, 724, 824).

5. Dispositif d'angioplastie transluminale percutanée (610, 710, 810) selon la revendication 4, dans lequel une traction sur l'extrémité proximale du fil d'actionnement (650, 750, 850) tire la partie annulaire distale (624, 724, 824) en direction de la partie annulaire proximale fixe (622, 722, 822).

6. Dispositif d'angioplastie transluminale percutanée (610, 710, 810) selon la revendication 1, dans lequel le dispositif d'intervention (18) comprend un ballonnet d'angioplastie (18).

7. Dispositif d'angioplastie transluminale percutanée (610, 710, 810) selon la revendication 1, dans lequel le dispositif d'intervention (18) comprend un stent.

8. Dispositif d'angioplastie transluminale percutanée (610, 710, 810) selon la revendication 1, dans lequel le dispositif d'intervention (18) comprend un dispositif de thrombectomie mécanique.

9. Dispositif d'angioplastie transluminale percutanée (610, 710, 810) selon la revendication 1, dans lequel le matériau à mémoire de forme comprend le Nitinol ou un alliage cobalt-chrome.

10. Dispositif d'angioplastie transluminale percutanée (610, 710, 810) selon la revendication 1, dans lequel la membrane de filtration (1700) recouvre une partie distale (1808) de la structure à mailles en fils tressés (1800).

11. Dispositif d'angioplastie transluminale percutanée (610, 710, 810) selon la revendication 1, dans lequel la membrane de filtration (1700) s'étend au-delà de la structure à mailles en fils tressés (1800) dans une direction longitudinale par rapport à l'axe longitudinal du cathéter (12), de telle sorte qu'un sac (1398) soit formé pour retenir des particules emboliques lorsque le filtre (620, 720, 820) se trouve dans la position comprimée.

12. Dispositif d'angioplastie transluminale percutanée (610, 710, 810) selon la revendication 1, dans lequel la structure à mailles en fils tressés (1800) comprend des fils en métal, des fils en polymère et similaires.

13. Dispositif d'angioplastie transluminale percutanée (610, 710, 810) selon la revendication 1, dans lequel la structure à mailles en fils tressés (1800) est formée à partir d'une tresse de fils comprenant entre environ 12 et environ 16 fils.

14. Dispositif d'angioplastie transluminale percutanée (610, 710, 810) selon la revendication 1, dans lequel un angle de tressage (1804) entre les fils de la structure à mailles en fils tressés (1800) et un axe longitudinal de la structure à mailles en fils tressés (1800) est un multiple, compris entre environ 1,5 fois et 4 fois, de l'angle entre le fil et l'axe médian lorsque le fil se trouve dans la position de référence fermée ou comprimée.

15. Dispositif d'angioplastie transluminale percutanée (610, 710, 810) selon l'une quelconque des revendications 1 à 14, dans lequel la structure à mailles en fils tressés (1800) est formée à partir de fils ronds présentant un diamètre de 60 micromètres et un angle de tressage, sur une tige de 7 mm, qui correspond au diamètre déployé maximal (1806) qui est le double (2 fois) de l'angle entre le fil et l'axe médian, l'angle de tressage étant éventuellement de 150°.

16. Dispositif d'angioplastie transluminale percutanée (610, 710, 810) selon l'une quelconque des revendications 1 à 15, dans lequel la structure à mailles en fils tressés (1800) forme une ouverture de maille large lorsqu'elle est ouverte de façon à permettre un écoulement sanguin dans la membrane de filtration (1700).
